# EUROPEAN PATENT SPECIFICATION

(11) **EP 3 080 256 B1**
(45) Date of publication and mention of the grant of the patent: **13.06.2018**
(21) Application number: 14812222.9
(22) Date of filing: 12.12.2014
(51) Int. Cl.: C12N 9/22, C12N 15/09

(54) **CAS9 NUCLEASE PLATFORM FOR MICROALGAE GENOME ENGINEERING**
CAS9-NUKLEASE-PLATTFORM ZUR MIKROALGENGENOMENGINEERING
PLATEFORME NUCLÉASE CAS9 POUR INGÉNIERIE GÉNOMIQUE DE MICRO-ALGUES

(30) Priority: 13.12.2013 DK 201370772
(43) Date of publication of application: 19.10.2016
(73) Proprietor: Cellectis, 75013 Paris (FR)
(72) Inventor: DABOUSSI, Fayza, F-77500 Chelles (FR); BEURDELEY, Marine, F-75009 Paris (FR); JUILLERAT, Alexandre, New York, NY 10028 (US)
(74) Representative: Sellin, Carole
(86) International application number: PCT/EP2014/077508
(87) International publication number: WO 2015/086795

(56) References cited:
- WO-A1-2013/176772
- WO-A2-2012/017329
- US-A1- 2013 065 314
- KHAOULA BELHAJ ET AL: "Plant genome editing made easy: targeted mutagenesis in model and crop plants using the CRISPR/Cas system", PLANT METHODS, BIOMED CENTRAL, LONDON, GB, vol. 9, no. 1, 11 October 2013 (2013-10-11), page 39, XP021164954, ISSN: 1746-4811, DOI: 10.1186/1746-4811-9-39
- VLADIMIR NEKRASOV ET AL: "Targeted mutagenesis in the model plant Nicotiana benthamiana using Cas9 RNA-guided endonuclease", NATURE BIOTECHNOLOGY, vol. 31, no. 8, 1 August 2013 (2013-08-01) , pages 691-693, XP055129106, ISSN: 1087-0156, DOI: 10.1038/nbt.2654

### SEQUENCE LISTING

<110> CELLECTIS
<120> CAS9 NUCLEASE PLATFORM FOR MICROALGAE GENOME ENGINEERING
<130> P81314600PCT00
<150> PA201370778
   <151> 2013-12-13
<160> 5
<170> PatentIn version 3.5
<210> 1
   <211> 1368
   <212> PRT
   <213> Streptococcus pyogenes serotype M1
<400> 1
<210> 2
   <211> 10
   <212> PRT
   <213> artificial sequence
<220>
   <223> RuvC motif
<220>
   <221> misc_feature
   <222> (2)..(2)
   <223> Xaa can be any naturally occurring amino acid
<220>
   <221> misc_feature
   <222> (4)..(5)
   <223> Xaa can be any naturally occurring amino acid
<220>
   <221> misc_feature
   <222> (7)..(7)
   <223> Xaa can be any naturally occurring amino acid
<400> 2
   Asp Xaa Gly Xaa Xaa Ser Xaa Gly Trp Ala
   1               5                   10
<210> 3
   <211> 16
   <212> PRT
   <213> artificial sequence
<220>
   <223> HNH motif
<220>
   <221> misc_feature
   <222> (2)..(3)
   <223> Xaa can be any naturally occurring amino acid
<220>
   <221> misc_feature
   <222> (6)..(7)
   <223> Xaa can be any naturally occurring amino acid
<220>
   <221> misc_feature
   <222> (9)..(9)
   <223> Xaa can be any naturally occurring amino acid
<220>
   <221> misc_feature
   <222> (11)..(13)
   <223> Xaa can be any naturally occurring amino acid
<220>
   <221> misc_feature
   <222> (15)..(15)
   <223> Xaa can be any naturally occurring amino acid
<400> 3
   Tyr Xaa Xaa Asp His Xaa Xaa Pro Xaa Ser Xaa Xaa Xaa Asp Xaa Ser
   1               5                   10                  15
<210> 4
   <211> 247
   <212> PRT
   <213> artificial sequence
<220>
   <223> Synthetic polypeptides: Split Cas9 RuvC
<400> 4
   Met Asp Lys Lys Tyr Ser Ile Gly Leu Asp Ile Gly Thr Asn Ser Val
   1               5                   10                  15
   Gly Trp Ala Val Ile Thr Asp Glu Tyr Lys Val Pro Ser Lys Lys Phe
               20                  25                  30
   Lys Val Leu Gly Asn Thr Asp Arg His Ser Ile Lys Lys Asn Leu Ile
           35                  40                  45
   Gly Ala Leu Leu Phe Asp Ser Gly Glu Thr Ala Glu Ala Thr Arg Leu
       50                  55                  60
   Lys Arg Thr Ala Arg Arg Arg Tyr Thr Arg Arg Lys Asn Arg Ile Cys
   65                  70                  75                  80
   Tyr Leu Gln Glu Ile Phe Ser Asn Glu Met Ala Lys Val Asp Asp Ser
                   85                  90                  95
   Phe Phe His Arg Leu Glu Glu Ser Phe Leu Val Glu Glu Asp Lys Lys
               100                 105                 110
   His Glu Arg His Pro Ile Phe Gly Asn Ile Val Asp Glu Val Ala Tyr
           115                 120                 125
   His Glu Lys Tyr Pro Thr Ile Tyr His Leu Arg Lys Lys Leu Val Asp
       130                 135                 140
   Ser Thr Asp Lys Ala Asp Leu Arg Leu Ile Tyr Leu Ala Leu Ala His
   145                 150                 155                 160
   Met Ile Lys Phe Arg Gly His Phe Leu Ile Glu Gly Asp Leu Asn Pro
                   165                 170                 175
   Asp Asn Ser Asp Val Asp Lys Leu Phe Ile Gln Leu Val Gln Thr Tyr
               180                 185                 190
   Asn Gln Leu Phe Glu Glu Asn Pro Ile Asn Ala Ser Gly Val Asp Ala
           195                 200                 205
   Lys Ala Ile Leu Ser Ala Arg Leu Ser Lys Ser Arg Arg Leu Glu Asn
       210                 215                 220
   Leu Ile Ala Gln Leu Pro Gly Glu Lys Lys Asn Gly Leu Phe Gly Asn
   225 230 235 240
   Leu Ile Ala Leu Ser Leu Gly
   245
<210> 5
   <211> 1121
   <212> PRT
   <213> artificial sequence
<220>
   <223> Synthetic polypeptides: Split Cas9 HNH
<400> 5
   Leu Thr Pro Asn Phe Lys Ser Asn Phe Asp Leu Ala Glu Asp Ala Lys
   1 5 10 15
   Leu Gln Leu Ser Lys Asp Thr Tyr Asp Asp Asp Leu Asp Asn Leu Leu
   20 25 30
   Ala Gln Ile Gly Asp Gln Tyr Ala Asp Leu Phe Leu Ala Ala Lys Asn
   35 40 45
   Leu Ser Asp Ala Ile Leu Leu Ser Asp Ile Leu Arg Val Asn Thr Glu
   50 55 60
   Ile Thr Lys Ala Pro Leu Ser Ala Ser Met Ile Lys Arg Tyr Asp Glu
   65 70 75 80
   His His Gln Asp Leu Thr Leu Leu Lys Ala Leu Val Arg Gln Gln Leu
   85 90 95
   Pro Glu Lys Tyr Lys Glu Ile Phe Phe Asp Gln Ser Lys Asn Gly Tyr
   100 105 110
   Ala Gly Tyr Ile Asp Gly Gly Ala Ser Gln Glu Glu Phe Tyr Lys Phe
   115 120 125
   Ile Lys Pro Ile Leu Glu Lys Met Asp Gly Thr Glu Glu Leu Leu Val
   130 135 140
   Lys Leu Asn Arg Glu Asp Leu Leu Arg Lys Gln Arg Thr Phe Asp Asn
   145 150 155 160
   Gly Ser Ile Pro His Gln Ile His Leu Gly Glu Leu His Ala Ile Leu
   165 170 175
   Arg Arg Gln Glu Asp Phe Tyr Pro Phe Leu Lys Asp Asn Arg Glu Lys
               180                 185                 190
   Ile Glu Lys Ile Leu Thr Phe Arg Ile Pro Tyr Tyr Val Gly Pro Leu
           195                 200                 205
   Ala Arg Gly Asn Ser Arg Phe Ala Trp Met Thr Arg Lys Ser Glu Glu
       210                 215                 220
   Thr Ile Thr Pro Trp Asn Phe Glu Glu Val Val Asp Lys Gly Ala Ser
   225                 230                 235                 240
   Ala Gln Ser Phe Ile Glu Arg Met Thr Asn Phe Asp Lys Asn Leu Pro
                   245                 250                 255
   Asn Glu Lys Val Leu Pro Lys His Ser Leu Leu Tyr Glu Tyr Phe Thr
               260                 265                 270
   Val Tyr Asn Glu Leu Thr Lys Val Lys Tyr Val Thr Glu Gly Met Arg
           275                 280                 285
   Lys Pro Ala Phe Leu Ser Gly Glu Gln Lys Lys Ala Ile Val Asp Leu
       290                 295                 300
   Leu Phe Lys Thr Asn Arg Lys Val Thr Val Lys Gln Leu Lys Glu Asp
   305                 310                 315                 320
   Tyr Phe Lys Lys Ile Glu Cys Phe Asp Ser Val Glu Ile Ser Gly Val
                   325                 330                 335
   Glu Asp Arg Phe Asn Ala Ser Leu Gly Thr Tyr His Asp Leu Leu Lys
               340                 345                 350
   Ile Ile Lys Asp Lys Asp Phe Leu Asp Asn Glu Glu Asn Glu Asp Ile
           355                 360                 365
   Leu Glu Asp Ile Val Leu Thr Leu Thr Leu Phe Glu Asp Arg Glu Met
       370                 375                 380
   Ile Glu Glu Arg Leu Lys Thr Tyr Ala His Leu Phe Asp Asp Lys Val
   385                 390                 395                 400
   Met Lys Gln Leu Lys Arg Arg Arg Tyr Thr Gly Trp Gly Arg Leu Ser
                   405                 410                 415
   Arg Lys Leu Ile Asn Gly Ile Arg Asp Lys Gln Ser Gly Lys Thr Ile
               420                 425                 430
   Leu Asp Phe Leu Lys Ser Asp Gly Phe Ala Asn Arg Asn Phe Met Gln
           435                 440                 445
   Leu Ile His Asp Asp Ser Leu Thr Phe Lys Glu Asp Ile Gln Lys Ala
       450                 455                 460
   Gln Val Ser Gly Gln Gly Asp Ser Leu His Glu His Ile Ala Asn Leu
   465                 470                 475                 480
   Ala Gly Ser Pro Ala Ile Lys Lys Gly Ile Leu Gln Thr Val Lys Val
                   485                 490                 495
   Val Asp Glu Leu Val Lys Val Met Gly Arg His Lys Pro Glu Asn Ile
               500                 505                 510
   Val Ile Glu Met Ala Arg Glu Asn Gln Thr Thr Gln Lys Gly Gln Lys
           515                 520                 525
   Asn Ser Arg Glu Arg Met Lys Arg Ile Glu Glu Gly Ile Lys Glu Leu
       530                 535                 540
   Gly Ser Gln Ile Leu Lys Glu His Pro Val Glu Asn Thr Gln Leu Gln
   545                 550                 555                 560
   Asn Glu Lys Leu Tyr Leu Tyr Tyr Leu Gln Asn Gly Arg Asp Met Tyr
                   565                 570                 575
   Val Asp Gln Glu Leu Asp Ile Asn Arg Leu Ser Asp Tyr Asp Val Asp
               580                 585                 590
   His Ile Val Pro Gln Ser Phe Leu Lys Asp Asp Ser Ile Asp Asn Lys
           595                 600                 605
   Val Leu Thr Arg Ser Asp Lys Asn Arg Gly Lys Ser Asp Asn Val Pro
       610                 615                 620
   Ser Glu Glu Val Val Lys Lys Met Lys Asn Tyr Trp Arg Gln Leu Leu
   625                 630                 635                 640
   Asn Ala Lys Leu Ile Thr Gln Arg Lys Phe Asp Asn Leu Thr Lys Ala
                   645                 650                 655
   Glu Arg Gly Gly Leu Ser Glu Leu Asp Lys Ala Gly Phe Ile Lys Arg
               660                 665                 670
   Gln Leu Val Glu Thr Arg Gln Ile Thr Lys His Val Ala Gln Ile Leu
   675 680 685
   Asp Ser Arg Met Asn Thr Lys Tyr Asp Glu Asn Asp Lys Leu Ile Arg
   690 695 700
   Glu Val Lys Val Ile Thr Leu Lys Ser Lys Leu Val Ser Asp Phe Arg
   705 710 715 720
   Lys Asp Phe Gln Phe Tyr Lys Val Arg Glu Ile Asn Asn Tyr His His
   725 730 735
   Ala His Asp Ala Tyr Leu Asn Ala Val Val Gly Thr Ala Leu Ile Lys
   740 745 750
   Lys Tyr Pro Lys Leu Glu Ser Glu Phe Val Tyr Gly Asp Tyr Lys Val
   755 760 765
   Tyr Asp Val Arg Lys Met Ile Ala Lys Ser Glu Gln Glu Ile Gly Lys
   770 775 780
   Ala Thr Ala Lys Tyr Phe Phe Tyr Ser Asn Ile Met Asn Phe Phe Lys
   785 790 795 800
   Thr Glu Ile Thr Leu Ala Asn Gly Glu Ile Arg Lys Arg Pro Leu Ile
   805 810 815
   Glu Thr Asn Gly Glu Thr Gly Glu Ile Val Trp Asp Lys Gly Arg Asp
   820 825 830
   Phe Ala Thr Val Arg Lys Val Leu Ser Met Pro Gln Val Asn Ile Val
   835 840 845
   Lys Lys Thr Glu Val Gln Thr Gly Gly Phe Ser Lys Glu Ser Ile Leu
   850 855 860
   Pro Lys Arg Asn Ser Asp Lys Leu Ile Ala Arg Lys Lys Asp Trp Asp
   865 870 875 880
   Pro Lys Lys Tyr Gly Gly Phe Asp Ser Pro Thr Val Ala Tyr Ser Val
   885 890 895
   Leu Val Val Ala Lys Val Glu Lys Gly Lys Ser Lys Lys Leu Lys Ser
   900 905 910
   Val Lys Glu Leu Leu Gly Ile Thr Ile Met Glu Arg Ser Ser Phe Glu
   915 920 925
   Lys Asn Pro Ile Asp Phe Leu Glu Ala Lys Gly Tyr Lys Glu Val Lys
       930                 935                 940
   Lys Asp Leu Ile Ile Lys Leu Pro Lys Tyr Ser Leu Phe Glu Leu Glu
   945                 950                 955                 960
   Asn Gly Arg Lys Arg Met Leu Ala Ser Ala Gly Glu Leu Gln Lys Gly
                   965                 970                 975
   Asn Glu Leu Ala Leu Pro Ser Lys Tyr Val Asn Phe Leu Tyr Leu Ala
               980                 985                 990
   Ser His Tyr Glu Lys Leu Lys Gly  Ser Pro Glu Asp Asn  Glu Gln Lys
           995                 1000                 1005
   Gln Leu  Phe Val Glu Gln His  Lys His Tyr Leu Asp  Glu Ile Ile
       1010                 1015                 1020
   Glu Gln  Ile Ser Glu Phe Ser  Lys Arg Val Ile Leu  Ala Asp Ala
       1025                 1030                 1035
   Asn Leu  Asp Lys Val Leu Ser  Ala Tyr Asn Lys His  Arg Asp Lys
       1040                 1045                 1050
   Pro Ile  Arg Glu Gln Ala Glu  Asn Ile Ile His Leu  Phe Thr Leu
       1055                 1060                 1065
   Thr Asn  Leu Gly Ala Pro Ala  Ala Phe Lys Tyr Phe  Asp Thr Thr
       1070                 1075                 1080
   Ile Asp  Arg Lys Arg Tyr Thr  Ser Thr Lys Glu Val  Leu Asp Ala
       1085                 1090                 1095
   Thr Leu  Ile His Gln Ser Ile  Thr Gly Leu Tyr Glu  Thr Arg Ile
       1100                 1105                 1110
   Asp Leu  Ser Gln Leu Gly Gly  Asp
       1115                 1120

## Description

### FIELD OF THE INVENTION

The present invention relates to a method of genome engineering in diatoms using the Cas9/CRISPR system. In particular, the present invention relates to methods of delivering guide RNA via cell penetrating peptides in diatoms, namely in stable integrated Cas9 diatoms. The present description also relates to kits and isolated cells comprising Cas9, split Cas9 or guide RNA and Cas9-fused cell-penetrating peptides. The present invention also relates to isolated cells obtained by the methods of the invention.

### BACKGROUND OF THE INVENTION

Diatoms represent a major group of photosynthetic microalgae, which has a vast potential for biotechnological purposes, in particular for oil production, but their spread is hampered by the lack of genetic manipulation tools. Indeed, although the genome of diatoms has now been sequenced, very few genetic tools are available at this time to explore their genetic diversity. As a first difficulty, diatoms remain difficult to transform by means of electroporation, probably due to their particular cell wall, which comprises a silica cytoskeleton. Biolistic methods remain the most common technique, but result into low survival rates. By using either of these techniques, transformants are present at very low frequencies, which makes gene editing tedious. As another difficulty, few genes are available to confer a resistance to the transformed cells by expression into selective culture media.

So far, the generation of strains with a modulated gene expression has laid mainly on the use of random gene over-expression and targeted gene-silencing system using RNA interference (RNAi) (Siaut, Heijde et al. 2007; De Riso, Raniello et al. 2009). In the past few years, new efficient tools for precise genome engineering have emerged in the field of plant and mammalian cells, such as the Meganucleases, Zinc Finger nucleases, TALE nucleases and more recently the RNA-guided Cas9 nucleases. This opened the path for using rare-cutting endonucleases for precise genome engineering into microalgae. But, to the inventor's knowledge, only meganucleases and TALE-nucleases have proven so far to induce targeted and stable genome modifications in diatoms (International application WO2012017329). For industrial purposes and safety reasons, it would be an advantage not to insert transgenes into the algae genomes when performing gene editing in algal cells. Transient expression of the endonucleases would be also advantageous to limit the risk of releasing genetically modified algae in the environment, which would include foreign genes in their genomes. Thus, new genetic tools for precise genome engineering are still desirable to explore and exploit the full genetic potential of microalgae.

The present inventors propose to use the Cas9 system as new method to induce precise gene modifications in diatoms. They used a biolistic transformation method to do a stable and targeted integration of the Cas9 protein and co-transfect its corresponding guide RNA into diatom cells.

Although such transformation method has proved to be effective in microalgae, it appears to show relatively weak efficiency with a frequency comprised between 10⁻⁸ and 10⁻⁶ thus requiring the introduction of an antibiotic selection such as nourseothricin or phleomycin to easily detect the clones (De Riso, Raniello et al. 2009). Another drawback of such transformation method is the delay of three to five weeks to obtain microalgae clones following transformation. Finally, the major drawback for this biolistic method is associated with the physical penetration of metal beads into the algae cells leading to deleterious effects for the cells (cell damage or contamination).

Considering these points and the fact that the delivery of biological or chemical cargoes have been restricted to physical and mechanical methods, mostly in cell wall-deficient mutants (Azencott, Peter et al. 2007; Kilian, Benemann et al. 2011), the inventors propose, as per the present invention, to enable Cas9/CRISPR complexes to penetrate the cell wall and the cell membrane of algae by using cell-penetrating peptides (CPP),- i.e. peptides which are rich in basic amino-acids and that can penetrate the cells -, in order to efficiently edit algae genomes.

### SUMMARY OF THE INVENTION

The inventors developed a new genome engineering method to transform Diatom cells based on the CRISPR/Cas9 system. In particular, the inventors propose to deliver RNA guides via a CPP fusion (CPP::guide RNA) into diatoms already stably transformed with the Cas9 nuclease. This invention can be of particular interest to easily do targeted multiplex gene modifications and to create an inducible nuclease system by adding or not the CPP::guide RNA to the Cas9 cells. The inventors also showed that Cas9 protein can be divided into two separate split Cas9 RuvC and HNH domains which can process target nucleic acid sequence together or separately with guide RNA. This Cas9 split system is particularly suitable for an inducible method of genome targeting and to avoid the potential toxic effect of the Cas9 overexpression within the cell. Indeed, a first split Cas9 domain can be introduced into the cell, preferably by stably transforming said cell with a transgene encoding said split domain. Then, the complementary split part of Cas9 can be introduced into the cell, such that the two split parts reassemble into the cell to reconstitute a functional Cas9 protein at the desired time. Moreover, the reduction of the size of the split Cas9 compared to wild type Cas9 ease the vectorization and the delivery into the cell, as example by using cell penetrating peptide.

The description also proposes to vectorize via a CPP fusion both the Cas9 protein or split Cas9 and its RNA guide thus avoiding the major drawbacks of conventional transformation methods in algae, such as weak transformation efficiency, long delay to obtain clones following transformation and deleterious effect due to the introduction of metal beads into the cells.

Generation of genetically modified diatoms will be improved in term of safety and efficacy by using this method, allowing specific gene mutagenesis and gene insertion within the diatom genome.

### DESCRIPTION OF THE INVENTION

The present invention relates to a method of genome engineering in diatoms, particularly based on the CRISPR/Cas system for various applications ranging from targeted nucleic acid cleavage to targeted gene regulation. This method derives from the genome engineering CRISPR adaptive immune system tool that has been developed based on the RNA-guided Cas9 nuclease (Gasiunas, Barrangou et al. 2012; Jinek, Chylinski et al. 2012).

In a particular embodiment, the present invention relates to a method of genome engineering diatoms using the cas9/CRISPR comprising:
(a) Selecting a target nucleic acid sequence, optionally comprising a PAM motif;
(b) Providing a diatom, wherein a transgene encoding Cas9 or at least one split Cas9 is stably integrated within the genome of said diatom;
(c) Providing at least one guide RNA comprising a complementary sequence to the target nucleic acid;
(d) Introducing into said diatom, at least one guide RNA such that said Cas9 or split Cas9 processes said target nucleic acid sequence;
wherein said guide RNA is fused to a cell-penetrating peptide, and said guide RNA is introduced into said diatom by contacting said diatom with the fusion guide RNA: cell-penetrating peptide.

According to an aspect, the description discloses a method of genome engineering diatoms comprising:
(a) selecting a target nucleic acid sequence, optionally comprising a PAM motif in diatom;
(b) providing a guide RNA comprising a sequence complementary to the target nucleic acid sequence
(c) providing a Cas9 protein;
(d) introducing into the cell said guide RNA and said Cas9, such that Cas9 processes the target nucleic acid sequence in the cell.

The term "process" as used herein means that sequence is considered modified simply by the binding of the Cas9. Depending of the Cas9 used, different processed event can be induced within the target nucleic acid sequence. As non limiting example, Cas9 can induce cleavage, nicking events or can yield to or specific activating, repressing or silencing of the gene of interest. Any target nucleic acid sequences can be processed by the present methods. The target nucleic acid sequence (or DNA target) can be present in a chromosome, an episome, an organellar genome such as mitochondrial or chloroplast genome or genetic material that can exist independently to the main body of genetic material such as an infecting viral genome, plasmids, episomes, transposons for example. A target nucleic acid sequence can be within the coding sequence of a gene, within transcribed non-coding sequence such as, for example, leader sequences, trailer sequence or introns, or within non-transcribed sequence, either upstream or downstream of the coding sequence. The nucleic acid target sequence is defined by the 5' to 3' sequence of one strand of said target.

### Cas9

Cas9, also named Csn1 (COG3513 - SEQ ID NO: 1) is a large protein that participates in both crRNA biogenesis and in the destruction of invading DNA. Cas9 has been described in different bacterial species such as *S*. *thermophilus* (Sapranauskas, Gasiunas et al. 2011), *listeria innocua* (Gasiunas, Barrangou et al. 2012; Jinek, Chylinski et al. 2012) and *S*. *Pyogenes* (Deltcheva, Chylinski et al. 2011). The large Cas9 protein (>1200 amino acids) contains two predicted nuclease domains, namely HNH (McrA-like) nuclease domain that is located in the middle of the protein and a splitted RuvC-like nuclease domain (RNase H fold) (Haft, Selengut et al. 2005; Makarova, Grishin et al. 2006).

By Cas9 is also meant an engineered endonuclease or a homologue of Cas9 which is capable of processing target nucleic acid sequence. In particular embodiment, Cas9 can induce a cleavage in the nucleic acid target sequence which can correspond to either a double-stranded break or a single-stranded break. Cas9 variant can be a Cas9 endonuclease that does not naturally exist in nature and that is obtained by protein engineering or by random mutagenesis. Cas9 variants according to the invention can for example be obtained by mutations i.e. deletions from, or insertions or substitutions of at least one residue in the amino acid sequence of a *S*. *pyogenes* Cas9 endonuclease (SEQ ID NO: 1). In the frame aspects of the present invention, such Cas9 variants remain functional, i.e. they retain the capacity of processing a target nucleic acid sequence. Cas9 variant can also be homologues of *S*. *pyogenes* Cas9 which can comprise deletions from, or insertions or substitutions of, at least one residue within the amino acid sequence of *S*. *pyogenes* Cas9 (SEQ ID NO: 1). Any combination of deletion, insertion, and substitution may also be made to arrive at the final construct, provided that the final construct possesses the desired activity, in particular the capacity of binding a guide RNA or nucleic acid target sequence.

RuvC/RNaseH motif includes proteins that show wide spectra of nucleolytic functions, acting both on RNA and DNA (RNaseH, RuvC, DNA transposases and retroviral integrases and PIWI domain of Argonaut proteins). In the present invention the RuvC catalytic domain of the Cas9 protein can be characterized by the sequence motif: D-[I/L]-G-X-X-S-X-G-W-A, wherein X represents any one of the natural 20 amino acids and [I/L] represents isoleucine or leucine (SEQ ID NO: 2). In other terms, the present invention relates to Cas9 variant which comprises at least D-[I/L]-G-X-X-S-X-G-W-A sequence, wherein X represents any one of the natural 20 amino acids and [I/L] represents isoleucine or leucine (SEQ ID NO: 2).

HNH motif is characteristic of many nucleases that act on double-stranded DNA including colicins, restriction enzymes and homing endonucleases. The domain HNH (SMART ID: SM00507, SCOP nomenclature:HNH family) is associated with a range of DNA binding proteins, performing a variety of binding and cutting functions (Gorbalenya 1994; Shub, Goodrich-Blair et al. 1994). Several of the proteins are hypothetical or putative proteins of no well-defined function. The ones with known function are involved in a range of cellular processes including bacterial toxicity, homing functions in groups I and II introns and inteins, recombination, developmentally controlled DNA rearrangement, phage packaging, and restriction endonuclease activity (Dalgaard, Klar et al. 1997). These proteins are found in viruses, archaebacteria, eubacteria, and eukaryotes. Interestingly, as with the LAGLI-DADG and the GIY-YIG motifs, the HNH motif is often associated with endonuclease domains of self-propagating elements like inteins, Group I, and Group II introns (Gorbalenya 1994; Dalgaard, Klar et al. 1997). The HNH domain can be characterized by the presence of a conserved Asp/His residue flanked by conserved His (amino-terminal) and His/Asp/Glu (carboxy-terminal) residues at some distance. A substantial number of these proteins can also have a CX2C motif on either side of the central Asp/His residue. Structurally, the HNH motif appears as a central hairpin of twisted β-strands, which are flanked on each side by an α helix (Kleanthous, Kuhlmann et al. 1999). In the present invention, the HNH motif can be characterized by the sequence motif: Y-X-X-D-H-X-X-P-X-S-X-X-X-D-X-S, wherein X represents any one of the natural 20 amino acids (SEQ ID NO: 3). The present invention relates to a Cas9 variant which comprises at least Y-X-X-D-H-X-X-P-X-S-X-X-X-D-X-S sequence wherein X represents any one of the natural 20 amino acids (SEQ ID NO: 3).

### Split Cas9 system

The previous characterization of the RuvC and HNH domains prompted the inventors to engineer Cas9 protein to create split Cas9 protein. Surprisingly, the inventors showed that these two split Cas9 could process together or separately the nucleic acid target. This observation allows developing a new Cas9 system using split Cas9 protein. Each split Cas9 domains can be prepared and used separately. Thus, this split system displays several advantages for vectorization, delivery methods in diatoms, allowing delivering shorter protein than the entire Cas9, and is particularly suitable to induce genome engineering in algae at the desired time and thus limiting the potential toxicity of an integrated Cas9 nuclease.

By "Split Cas9" is meant here a reduced or truncated form of a Cas9 protein or Cas9 variant, which comprises either a RuvC or HNH domain, but not both of these domains. Such "Split Cas9" can be used independently with guide RNA or in a complementary fashion, like for instance, one Split Cas9 providing a RuvC domain and another providing the HNH domain. Different split Cas9 may be used together having either RuvC and/or NHN domains.

RuvC domain generally comprises at least an amino acid sequence D-[I/L]-G-X-X-S-X-G-W-A, wherein X represents any one of the natural 20 amino acids and [I/L] represents isoleucine or leucine (SEQ ID NO: 2). HNH domain generally comprises at least an amino acid sequence Y-X-X-D-H-X-X-P-X-S-X-X-X-D-X-S sequence, wherein X represents any one of the natural 20 amino acids (SEQ ID NO: 3). More preferably said split domain comprising a RuvC domain comprises an amino acid sequence SEQ ID NO: 4. Said split domain comprising an HNH domain comprises an amino acid sequence SEQ ID NO: 5. In a preferred embodiment, said HNH domain comprises a first amino acid Leucine mutated in Valine in SEQ ID NO: 5 to have a better kozak consensus sequence.

Each Cas9 split domain can be derived from different Cas9 homologues, or can be derived from the same Cas9.

In particular, said method of genome engineering comprises:
(a) selecting a target nucleic acid sequence, optionally comprising a PAM motif in the cell;
(b) providing a guide RNA comprising a sequence complementary to the target nucleic acid sequence;
(c) providing at least one split Cas9 domain;
(d) introducing into the cell the guide RNA and said split Cas9 domain(s), such that split Cas9 domain(s) processes the target nucleic acid sequence in the cell.

Said Cas9 split domains (RuvC and HNH domains) can be simultaneously or sequentially introduced into the cell such that said split Cas9 domain(s) process the target nucleic acid sequence in the cell. Said Cas9 split domains and guide RNA can be introduced into the cell by using cell penetrating peptides as described below. This method is particularly suitable to generate no genetically modified algae.

The Cas9 split system is particularly suitable for an inducible method of genome targeting. In a preferred embodiment, to avoid the potential toxic effect of the Cas9 over expression due to its integration within the genome of a cell, a split Cas9 domain is introduced into the cell, by stably transforming said cell with a transgene encoding said split domain. Then, the complementary split part of Cas9 is introduced into the cell, such that the two split parts reassemble into the cell to reconstitute a functional Cas9 protein at the desired time. Said split Cas9 can be derived from the same Cas9 protein or can be derived from different Cas9 variants, particularly RuvC and HNH domains as described above.

In another aspect of the invention, only one split Cas9 domain is introduced into said cell. Indeed, surprisingly the inventors showed that the split Cas9 domain comprising the RuvC motif as described above is capable of cleaving a target nucleic acid sequence independently of split domain comprising the HNH motif. The guide RNA does not need the presence of the HNH domain to bind to the target nucleic acid sequence and is sufficiently stable to be bound by the RuvC split domain. In a preferred embodiment, said split Cas9 domain alone is capable of nicking said target nucleic acid sequence.

In another particular embodiment, potential endogenous RuvC and/or HNH catalytic domain can be encoded by the algae genome. Thus, endogenous RuvC and/or HNN expression can be able to process target nucleic acid sequence in presence of guideRNA. The present method can comprise the step of selecting a target nucleic acid sequence, optionally comprising a PAM motif, providing a guide RNA comprising a sequence complementary to the target nucleic acid sequence, providing a split Cas9 domain and introducing into the cell said complementary nucleic acid, optionally with said split Cas9 domain to process the target nucleic acid sequence.

Each split domain can be fused to at least one active domain in the N-terminal and/or C-terminal end, said active domain can be selected from the group consisting of: nuclease (e.g. endonuclease or exonuclease), polymerase, kinase, phosphatase, methylase, demethylase, acetylase, desacetylase, topoisomerase, integrase, transposase, ligase, helicase, recombinase, transcriptional activator(e.g. VP64, VP16), transcriptional inhibitor (e.g; KRAB), DNA end processing enzyme (e.g. Trex2, Tdt), reporter molecule (e.g. fluorescent proteins, lacZ, luciferase).

HNH domain is responsible for nicking of one strand of the target double-stranded DNA and the RuvC-like RNaseH fold domain is involved in nicking of the other strand (comprising the PAM motif) of the double-stranded nucleic acid target (Jinek, Chylinski et al. 2012). However, in wild-type Cas9, these two domains result in blunt cleavage of the invasive DNA within the same target sequence (proto-spacer) in the immediate vicinity of the PAM (Jinek, Chylinski et al. 2012). Cas 9 can be a nickase and induces a nick event within different target sequences. As non-limiting example, Cas9 or split Cas9 can comprise mutation(s) in the catalytic residues of either the HNH or RuvC-like domains, to induce a nick event within different target sequences. As non-limiting example, the catalytic residues of the Cas9 protein are those corresponding to amino acids D10, D31, H840, H868, N882 and N891 of SEQ ID NO: 1 or aligned positions using CLUSTALW method on homologues of Cas Family members. Any of these residues can be replaced by any other amino acids, preferably by alanine residue. Mutation in the catalytic residues means either substitution by another amino acids, or deletion or addition of amino acids that induce the inactivation of at least one of the catalytic domain of cas9. (cf (Sapranauskas, Gasiunas et al. 2011; Jinek, Chylinski et al. 2012). In a particular embodiment, Cas9 or split Cas9 may comprise one or several of the above mutations. In another particular embodiment, split Cas9 comprises only one of the two RuvC and HNH catalytic domains. In the present invention, Cas9 of different species, Cas9 homologues, Cas9 engineered and functional variant thereof can be used. The invention envisions the use of such Cas9 or split Cas9 variants to perform nucleic acid cleavage in a genetic sequence of interest. Said Cas9 or split Cas9 variants have an amino acid sequence sharing at least 70 %, preferably at least 80 %, more preferably at least 90%, and even more preferably 95 % identity with Cas9 of different species, Cas9 homologues, Cas9 engineered and functional variant thereof. Preferably, said Cas9 variants have an amino acid sequence sharing at least 70 %, preferably at least 80 %, more preferably at least 90%, and even more preferably 95 % identity with SEQ ID NO: 1.

In another aspect of the present invention, Cas9 or split Cas9 lacks endonucleolytic activity. The resulting Cas9 or split Cas9 is co-expressed with guide RNA designed to comprises a complementary sequence of the target nucleic acid sequence. Expression of Cas9 lacking endonucleolytic activity yields to specific silencing of the gene of interest. This system is named CRISPR interference (CRISPRi) (Qi, Larson et al. 2013). By silencing, it is meant that the gene of interest is not expressed in a functional protein form. The silencing may occur at the transcriptional or the translational step. According to the present invention, the silencing may occur by directly blocking transcription, more particularly by blocking transcription elongation or by targeting key cis-acting motifs within any promoter, sterically blocking the association of their cognate trans-acting transcription factors. The Cas9 lacking endonucleolytic activity comprises both non-functional HNH and RuvC domains. In particular, the Cas9 or split Cas9 polypeptide comprises inactivating mutations in the catalytic residues of both the RuvC-like and HNH domains. For example, the catalytic residues required for cleavage Cas9 activity can be the D10, D31, H840, H865, H868, N882 and N891 of SEQ ID NO: 1 or aligned positions using CLUSTALW method on homologues of Cas Family members. The residues comprised in HNH or RuvC motifs can be those described in the above paragraph. Any of these residues can be replaced by any one of the other amino acids, preferably by alanine residue. Mutation in the catalytic residues means either substitution by another amino acids, or deletion or addition of amino acids that induce the inactivation of at least one of the catalytic domain of cas9.

In another particular embodiment, Cas9 or each split domains can be fused to at least one active domain in the N-terminal and/or C-terminal end. Said active domain can be selected from the group consisting of: nuclease (e.g. endonuclease or exonuclease), polymerase, kinase, phosphatase, methylase, demethylase, acetylase, desacetylase, topoisomerase, integrase, transposase, ligase, helicase, recombinase, transcriptional activator(e.g. VP64, VP16), transcriptional inhibitor (e.g; KRAB), DNA end processing enzyme (e.g. Trex2, Tdt), reporter molecule (e.g. fluorescent proteins, lacZ, luciferase).

### PAM motif

Any potential selected target nucleic acid sequence in the present invention may have a specific sequence on its 3' end, named the protospacer adjacent motif or protospacer associated motif (PAM). The PAM is present in the targeted nucleic acid sequence but not in the crRNA that is produced to target it. Preferably, the proto-spacer adjacent motif (PAM) may correspond to 2 to 5 nucleotides starting immediately or in the vicinity of the proto-spacer at the leader distal end. The sequence and the location of the PAM vary among the different systems. PAM motif can be for examples NNAGAA, NAG, NGG, NGGNG, AWG, CC, CC, CCN, TCN, TTC as non limiting examples (shah SA, RNA biology 2013). Different Type II systems have differing PAM requirements. For example, the *S*. *pyogenes* system requires an NGG sequence, where N can be any nucleotides. *S*. *thermophilus* Type II systems require NGGNG (Horvath and Barrangou 2010) and NNAGAAW (Deveau, Barrangou et al. 2008), while different *S*. *mutant* systems tolerate NGG or NAAR (van der Ploeg 2009). PAM is not restricted to the region adjacent to the proto-spacer but can also be part of the proto-spacer (Mojica, Diez-Villasenor et al. 2009). In a particular embodiment, the Cas9 protein can be engineered not to recognize any PAM motif or to recognize a non natural PAM motif. In this case, the selected target sequence may comprise a smaller or a larger PAM motif with any combinations of amino acids. In a preferred embodiment, the selected target sequence comprise a PAM motif which comprises at least 3, preferably, 4, more preferably 5 nucleotides recognized by the Cas9 variant according to the present invention.

### Guide RNA

The method of the present invention comprises providing an engineered guide RNA. Guide RNA corresponds to a nucleic acid sequence comprising a complementary sequence. Preferably, said guide RNA correspond to a crRNA and tracrRNA which can be used separately or fused together.

In natural type II CRISPR system, the CRISPR targeting RNA (crRNA) targeting sequences are transcribed from DNA sequences known as protospacers. Protospacers are clustered in the bacterial genome in a group called a CRISPR array. The protospacers are short sequences (∼20bp) of known foreign DNA separated by a short palindromic repeat and kept like a record against future encounters. To create the crRNA, the CRISPR array is transcribed and the RNA is processed to separate the individual recognition sequences between the repeats. The spacer-containing CRISPR locus is transcribed in a long pre-crRNA. The processing of the CRISPR array transcript (pre-crRNA) into individual crRNAs is dependent on the presence of a trans-activating crRNA (tracrRNA) that has sequence complementary to the palindromic repeat. The tracrRNA hybridizes to the repeat regions separating the spacers of the pre-crRNA, initiating dsRNA cleavage by endogenous RNase III, which is followed by a second cleavage event within each spacer by Cas9, producing mature crRNAs that remain associated with the tracrRNA and Cas9 and form the Cas9-tracrRNA:crRNA complex. Engineered crRNA with tracrRNA is capable of targeting a selected nucleic acid sequence, obviating the need of RNase III and the crRNA processing in general (Jinek, Chylinski et al. 2012).

In the present invention, crRNA is engineered to comprise a sequence complementary to a portion of a target nucleic acid such that it is capable of targeting, preferably cleaving the target nucleic acid sequence. In a particular embodiment, the crRNA comprises a sequence of 5 to 50 nucleotides, preferably 12 nucleotides which is complementary to the target nucleic acid sequence. In a more particular embodiment, the crRNA is a sequence of at least 30 nucleotides which comprises at least 10 nucleotides, preferably 12 nucleotides complementary to the target nucleic acid sequence.

In another aspect, crRNA can be engineered to comprise a larger sequence complementary to a target nucleic acid. Indeed, the inventors showed that the RuvC split Cas9 domain is able to cleave the target nucleic acid sequence only with a guide RNA. Thus, the guide RNA can bind the target nucleic acid sequence in absence of the HNH split Cas9 domain. The crRNA can be designed to comprise a larger complementary sequence, preferably more than 20 bp, to increase the annealing between DNA-RNA duplex without the need to have the stability effect of the HNH split domain binding. Thus, the crRNA can comprise a complementary sequence to a target nucleic acid sequence of more than 20 bp. Such crRNA allow increasing the specificity of the Cas9 activity.

The crRNA may also comprise a complementary sequence followed by 4-10 nucleotides on the 5'end to improve the efficiency of targeting (Cong, Ran et al. 2013; Mali, Yang et al. 2013). In preferred embodiment, the complementary sequence of the crRNA is followed in 3'end by a nucleic acid sequence named repeat sequences or 3'extension sequence.

Coexpression of several crRNA with distinct complementary regions to two different genes targeted both genes can be used simultaneously. Thus, in particular embodiment, the crRNA can be engineered to recognize different target nucleic acid sequences simultaneously. In this case, same crRNA comprises at least two distinct sequences complementary to a portion of the different target nucleic acid sequences. In a preferred embodiment, said complementary sequences are spaced by a repeat sequence.

The crRNA according to the present invention can also be modified to increase its stability of the secondary structure and/or its binding affinity for Cas9. In a particular embodiment, the crRNA can comprise a 2', 3'-cyclic phosphate. The 2', 3'- cyclic phosphate terminus seems to be involved in many cellular processes i.e. tRNA splicing, endonucleolytic cleavage by several ribonucleases, in self-cleavage by RNA ribozyme and in response to various cellular stress including accumulation of unfolded protein in the endoplasmatic reticulum and oxidative stress (Schutz, Hesselberth et al. 2010). The inventors have speculated that the 2', 3'-cyclic phosphate enhances the crRNA stability or its affinity/specificity for Cas9. Thus, the present invention relates to the modified crRNA comprising a 2', 3'-cyclic phosphate, and the methods for genome engineering based on the CRISPR/cas system (Jinek, Chylinski et al. 2012; Cong, Ran et al. 2013; Mali, Yang et al. 2013) using the modified crRNA.

The guide RNA may also comprise a Trans-activating CRISPR RNA (TracrRNA). Trans-activating CRISPR RNA according to the present invention are characterized by an anti-repeat sequence capable of base-pairing with at least a part of the 3' extension sequence of crRNA to form a tracrRNA:crRNA also named guide RNA (gRNA). TracrRNA comprises a sequence complementary to a region of the crRNA. A guide RNA comprising a fusion of crRNA and tracrRNA that forms a hairpin that mimics the tracrRNA-crRNA complex (Jinek, Chylinski et al. 2012; Cong, Ran et al. 2013; Mali, Yang et al. 2013) can be used to direct Cas9 endonuclease-mediated cleavage of target nucleic acid. The guide RNA may comprise two distinct sequences complementary to a portion of the two target nucleic acid sequences, preferably spaced by a repeat sequence.

In a particular embodiment, Cas9 according to the present invention can induce genetic modification resulting from a cleavage event in the target nucleic acid sequence that is commonly repaired through non-homologous end joining (NHEJ). NHEJ comprises at least two different processes. Mechanisms involve rejoining of what remains of the two DNA ends through direct re-ligation (Critchlow and Jackson 1998) or via the so-called microhomology-mediated end joining (Ma, Kim et al. 2003). Repair via non-homologous end joining (NHEJ) often results in small insertions or deletions and can be used for the creation of specific gene knockouts. By "cleavage event" is intended a double-strand break or a single-strand break event. Said modification may be a deletion of the genetic material, insertion of nucleotides in the genetic material or a combination of both deletion and insertion of nucleotides.

The present description also relates to a method for modifying target nucleic acid sequence further comprising the step of expressing an additional catalytic domain into a host cell. In an aspect, the present description relates to a method to increase mutagenesis wherein said additional catalytic domain is a DNA end-processing enzyme. Non limiting examples of DNA end-processing enzymes include 5-3' exonucleases, 3-5' exonucleases, 5-3' alkaline exonucleases, 5' flap endonucleases, helicases, hosphatase, hydrolases and template-independent DNA polymerases. Non limiting examples of such catalytic domain comprise of a protein domain or catalytically active derivate of the protein domain seleced from the group consisting of hExol (EXO1_HUMAN), Yeast Exol (EXO1_YEAST), E.coli Exol, Human TREX2, Mouse TREX1, Human TREX1, Bovine TREX1, Rat TREX1, TdT (terminal deoxynucleotidyl transferase) Human DNA2, Yeast DNA2 (DNA2_YEAST). In an aspect, said additional catalytic domain has a 3'-5'-exonuclease activity, and in a preferred aspect, said additional catalytic domain has TREX exonuclease activity, more preferably TREX2 activity. In another aspect, said catalytic domain is encoded by a single chain TREX polypeptide. Said additional catalytic domain may be fused to a nuclease fusion protein or chimeric protein according to the invention optionally by a peptide linker.

Endonucleolytic breaks are known to stimulate the rate of homologous recombination. Therefore, in another aspect, the present description relates to a method for inducing homologous gene targeting in the nucleic acid target sequence further comprising providing to the cell an exogeneous nucleic acid comprising at least a sequence homologous to a portion of the target nucleic acid sequence, such that homologous recombination occurs between the target nucleic acid sequence and the exogeneous nucleic acid.

In particular embodiments, said exogenous nucleic acid comprises first and second portions which are homologous to region 5' and 3' of the target nucleic acid sequence, respectively. Said exogenous nucleic acid in these embodiments also comprises a third portion positioned between the first and the second portion which comprises no homology with the regions 5' and 3' of the target nucleic acid sequence. Following cleavage of the target nucleic acid sequence, a homologous recombination event is stimulated between the target nucleic acid sequence and the exogenous nucleic acid. Preferably, homologous sequences of at least 50 bp, preferably more than 100 bp and more preferably more than 200 bp are used within said donor matrix. Therefore, the homologous sequence is preferably from 200 bp to 6000 bp, more preferably from 1000 bp to 2000 bp. Indeed, shared nucleic acid homologies are located in regions flanking upstream and downstream the site of the break and the nucleic acid sequence to be introduced should be located between the two arms.

Depending on the location of the target nucleic acid sequence wherein break event has occurred, such exogenous nucleic acid can be used to knock-out a gene, e.g. when exogenous nucleic acid is located within the open reading frame of said gene, or to introduce new sequences or genes of interest. Sequence insertions by using such exogenous nucleic acid can be used to modify a targeted existing gene, by correction or replacement of said gene (allele swap as a non-limiting example), or to up- or down-regulate the expression of the targeted gene (promoter swap as non-limiting example), said targeted gene correction or replacement.

### Selection markers

In a particular embodiment, the target nucleic acid sequence according to the present invention is a selectable marker gene which confers resistance to a toxic substrate to select transformed algae. Selectable markers according to the present invention serve to eliminate unwanted elements. In particular, selectable marker gene is an endogenous gene which confers sensitivity to medium comprising a toxic substrate. Thus, inactivation of the selectable marker gene confers resistance to medium comprising toxic substrate. These markers are often toxic or otherwise inhibitory to replication under certain conditions. Consequently, it is possible to select cell comprising inactivated selectable marker gene. Selection of cells can also be obtained through the use of strains auxotropic for a particular metabolite. A point mutation or deletion in a gene required for amino acid synthesis or carbon source metabolism as non limiting examples can be used to select against strains when grown on media lacking the required nutrient. In most cases a defined "minimal" media is required for selection. There are a number of selective auxotropic markers that can be used in rich media, such as *thyA* and *dapA-E* from *E. coli.*

As non limiting examples, said selectable markers can be the *tetAR* gene which confers resistance to tetracycline but sensitivity to lipophilic component such as fusaric and quinalic acids (Bochner, Huang et al. 1980; Maloy and Nunn 1981), *sacB b. subtilis* gene encoding levansucrase that converts sucrose to levans which is harmful to the bacteria (Steinmetz, Le Coq et al. 1983; Gay, Le Coq et al. 1985), *rpsL* gene encoding the ribosomal subunit protein (S12) target of streptomycin (Dean 1981), *ccdB* encoding a cell-killing protein which is a potent poison of bacterial gyrase (Bernard, Gabant et al. 1994), *PheS* encoding the alpha subunits of the Phe-tRNA synthetase, which renders bacteria sensitive to p-chlorophenylalanine (Kast 1994), a phenylalanine analog, *thya* gene encoding a Thymidine synthetase which confers sensitivity to trimethoprim and related compounds (Stacey and Simson 1965), *lacY* encoding lactose permease, which renders bacteria sensitive to t-o-nitrophenyl-β-D-galactopyranoside (Murphy, Stewart et al. 1995), the *amiE* gene encoding a protein which converts fluoroacetamide to the toxic compound fluoroacetate (Collier, Spence et al. 2001), *mazF* gene, thymidine kinase, the Uridine 5'-monophosphate synthase gene (UMPS) encoding a protein which is involved in de novo synthesis of pyrimidine nucleotides and conversion of 5-Fluoroorotic acid (5-FOA) into the toxic compound 5-fluorouracil leading to cell death (Sakaguchi, Nakajima et al. 2011), the nitrate reductase gene encoding a protein which confers sensitivity to chlorate (Daboussi, Djeballi et al. 1989), the tryptophane synthase gene which converts the indole analog 5-fluoroindole (5-FI) into the toxic tryptophan analog 5-fluorotryptophan (Rohr, Sarkar et al. 2004; Falciatore, Merendino et al. 2005). According to the present invention, said selectable marker can be homologous sequences of the different genes described above. Here, homology between protein or DNA sequences is defined in terms of shared ancestry. Two segments of DNA can have shared ancestry because of either a speciation event (orthologs) or a duplication event (paralogs). According to the invention, said cell is an algal cell, more specifically a diatom and said selectable marker genes is UMPS or nitrate reductase gene.

### Delivery methods

The methods of the invention involve introducing molecule of interest such as guide RNA (crRNA, tracrRNa, or fusion guide RNA), split Cas9, Cas9, exogenous nucleic acid, DNA end-processing enzyme into a cell. Guide RNA, split Cas9, Cas9, exogenous nucleic acid, DNA end-processing enzyme or others molecules of interest may be synthesized *in situ* in the cell as a result of the introduction of polynucleotide, preferably transgene comprised in vector encoding RNA or polypeptides into the cell. Alternatively, the molecule of interest could be produced outside the cell and then introduced thereto.

Said polynucleotide can be introduced into cell by, for example without limitation, electroporation, magnetophoresis. The latter is a nucleic acid introduction technology using the processes of magnetophoresis and nanotechnology fabrication of micro-sized linear magnets (Kuehnle et al., U. S. Patent No. 6,706,394; 2004; Kuehnle et al., U. S. Patent No. 5,516,670; 1996) that proved amenable to effective chloroplast engineering in freshwater *Chlamydomonas,* improving plastid transformation efficiency by two orders of magnitude over the state-of the-art of biolistics (Champagne et al., Magnetophoresis for pathway engineering in green cells. Metabolic engineering V: Genome to Product, Engineering Conferences International Lake Tahoe CA, Abstracts pp 76; 2004). Polyethylene glycol treatment of protoplasts is another technique that can be used to transform cells (Maliga 2004). In various embodiments, the transformation methods can be coupled with one or more methods for visualization or quantification of nucleic acid introduction into cell. Also appropriate mixtures commercially available for protein transfection can be used to introduce protein in algae. More broadly, any means known in the art to allow delivery inside cells or subcellular compartments of agents/chemicals and molecules (proteins) can be used including liposomal delivery means, polymeric carriers, chemical carriers, lipoplexes, polyplexes, dendrimers, nanoparticles, emulsion, natural endocytosis or phagocytose pathway as non-limiting examples. Direct introduction, such as microinjection of protein of interest in cell can be considered. In a more preferred embodiment, said transformation construct is introduced into host cell by particle inflow gun bombardment or electroporation.

### Cell-penetrating peptides delivery method

According to the invention, at least the guide RNA, and possibly others molecules of interest (named cargo molecule) is introduced into the cell by using cell penetrating peptides (CPP). In particular, the method may comprise a step of preparing composition comprising a cell penetrating peptide and a molecule of interest (named cargo molecule) and contacting the diatom to the composition. Said cargo molecule can be mixed with the cell penetrating peptide. Said CPP, preferably N-terminal or C-terminal end of CPP can also be associated with the cargo molecule. This association can be covalent or non-covalent. CPPs can be subdivided into two main classes, the first requiring chemical linkage with the cargo and the second involving the formation of stable, non-covalent complexes. Covalent bonded CPPs form a covalent conjugate with the cargo molecule by chemical cross-linking (e.g. disulfide bond) or by cloning followed by expression of a CPP fusion protein. In a preferred embodiment, said CPP bears a pyrydil disulfide function such that the thiol modified cargo molecule forms a disulfide bond with the CPP. Said disulfide bond can be cleaved in particular in a reducing environment such as cytoplasm. Non-covalent bonded CPPs are preferentially amphipathic peptide such as for examples pep-1 and MPG which can form stable complexes with cargo molecule through non covalent electrostatic and hydrophobic interactions.

Although definition of CPPs is constantly evolving, they are generally described as short peptides of less than 35 amino acids either derived from proteins or from chimeric sequences which are capable of transporting polar hydrophilic biomolecules across cell membrane in a receptor independent manner. CPP can be cationic peptides, peptides having hydrophobic sequences, amphipatic peptides, peptides having proline-rich and anti-microbial sequence, and chimeric or bipartite peptides (Pooga and Langel 2005). In a particular embodiment, cationic CPP can comprise multiple basic of cationic CPPs (e.g., arginine and/or lysine). Preferably, CCP are amphipathic and possess a net positive charge. CPPs are able to penetrate biological membranes, to trigger the movement of various biomolecules across cell membranes into the cytoplasm and to improve their intracellular routing, thereby facilitating interactions with the target. Examples of CPP can include: Tat, a nuclear transcriptional activator protein which is a 101 amino acid protein required for viral replication by human immunodeficiency virus type 1 (HIV-1), penetratin, which corresponds to the third helix of the homeoprotein Antennapedia in *Drosophilia*, Kaposi fibroblast growth factor (FGF) signal peptide sequence, integrin β3 signal peptide sequence; Guanine rich-molecular transporters, MPG, pep-1, sweet arrow peptide, dermaseptins, transportan, pVEC, Human calcitonin, mouse prion protein (mPrPr), polyarginine peptide Args sequence, VP22 protein from Herpes Simplex Virus, antimicrobial peptides Buforin I and SynB (REF: US2013/0065314). New variants of CPPs can combine different transduction domains.

In a preferred embodiment, said CPP can be fused covalently or no-covalently to cationic or liposomal polymers, such as polyethylenimine (PEI). In another preferred embodiment, to ease cargo molecules delivery, the cell wall or cell membrane permeability can be increased. The cell wall or membrane permeability can be increased by for example using polysaccharides-lyases or oligosaccharides-lyases which degrade the extracellular matrix enwrapping the microalgae cells. Said lyases can be heparinase, heparatinase, chondroitinase, hyaluronidase, glucuronase, endoH, PNGase, exo-α-D-mannosidase. Warm water treatment cell can also be realized at 30°C or 60°C to said algae in order to weaken the membrane or cell wall integrity of algae. In another preferred embodiment, the chloroquine drug can be used to improve the release of molecule, particularly endocytosed CPP-fused cargo molecules from endosomal vesicles into the cytosol.

In a particular embodiment, said cell penetrating peptide is linked (i.e. fused, covalently or no covalently-bound) to a reporter marker to select transformed cells. A reporter marker is one whose transcription is detectable and/or which expresses a protein which is also detectable, either of which can be assayed. Examples of readily detectable proteins include, β-galactosidase, fluorescent protein (e.g. green fluorescent protein (GFP), red, cyan, yellow fluorescent proteins, fluorescein, phycoerythrine), chemiluminescent protein, a radioisotope, a tag marker (e.g. HA, FLAG, fluorescein tag), luciferase, beta-galactosidase, beta lactamase, alkaline phosphatase and chloramphenicol acetyl transferase as well as enzymes or proteins, i.e. selectable markers, involved in nutrient biosynthesis such as Leu2, His3, Trp1, Lys2, Ade2 and Ura3.

### Isolated cells

In another aspect, the present invention relates to an isolated cell obtainable or obtained by the method described above. In particular, the present invention relates to a cell, namely a diatom, which comprises a Cas9 or split Cas9 stably integrated within its genome. In another particular embodiment, the present invention relates to an isolated cell comprising a cell-penetrating peptide fused to a guide RNA, a Cas9 or a split Cas9.

In the frame of the present description, "algae" or "algae cells" refer to different species of algae that can be used as host for selection method using nuclease of the present invention. Algae are mainly photoautotrophs unified primarily by their lack of roots, leaves and other organs that characterize higher plants. Term "algae" groups, without limitation, several eukaryotic phyla, including the Rhodophyta (red algae), Chlorophyta (green algae), Phaeophyta (brown algae), Bacillariophyta (diatoms), Eustigmatophyta and dinoflagellates as well as the prokaryotic phylum Cyanobacteria (blue-green algae). The term "algae" includes for example algae selected from : *Amphora, Anabaena*, *Anikstrodesmis*, *Botryococcus, Chaetoceros, Chlamydomonas, Chlorella*, *Chlorococcum, Cyclotella*, *Cylindrotheca*, *Dunaliella*, *Emiliana*, *Euglena*, *Hematococcus, Isochrysis, Monochrysis*, *Monoraphidium, Nannochloris, Nannnochloropsis*, *Navicula*, *Nephrochloris*, *Nephroselmis*, *Nitzschia*, *Nodularia*, *Nostoc, Oochromonas*, *Oocystis, Oscillartoria*, *Pavlova*, *Phaeodactylum, Playtmonas*, *Pleurochrysis*, *Porhyra*, *Pseudoanabaena*, *Pyramimonas, Stichococcus, Synechococcus, Synechocystis, Tetraselmis, Thalassiosira*, and *Trichodesmium.*

According to the invention, algae are specifically diatoms. Diatoms are unicellular phototrophs identified by their species-specific morphology of their amorphous silica cell wall, which vary from each other at the nanometer scale. Diatoms includes as non limiting examples: *Phaeodactylum, Fragilariopsis*, *Thalassiosira*, *Coscinodiscus, Arachnoidiscusm*, *Aster omphalus, Navicula*, *Chaetoceros*, *Chorethron, Cylindrotheca fusiformis*, *Cyclotella*, *Lampriscus, Gyrosigma*, *Achnanthes, Cocconeis, Nitzschia*, *Amphora, schyzochytrium and Odontella.* In a more preferred embodiment, diatoms according to the invention are from the species: *Thalassiosira pseudonana* or *Phaeodactylum tricornutum.*

### Kits

Another aspect of the description is a kit for algal cell selection comprising a cell penetrating peptide fused to a cargo molecule, preferably a Cas9, split Cas9 or a guide RNA which is specifically engineered to recognize a target nucleic acid sequence. The kit may further comprise one or several components required to realize the selection method as described above.

### DEFINITIONS

In the description above, a number of terms are used extensively. The following definitions are provided to facilitate understanding of the present embodiments.

Amino acid residues in a polypeptide sequence are designated herein according to the one-letter code, in which, for example, Q means Gin or Glutamine residue, R means Arg or Arginine residue and D means Asp or Aspartic acid residue.

Amino acid substitution means the replacement of one amino acid residue with another, for instance the replacement of an Arginine residue with a Glutamine residue in a peptide sequence is an amino acid substitution.

Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.

As used herein, "nucleic acid" or polynucleotide" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.

By "complementary sequence" is meant the sequence part of polynucleotide (e.g. part of crRNa or tracRNA) that can hybridize to another part of polynucleotides (e.g. the target nucleic acid sequence or the crRNA respectively) under standard low stringent conditions. Such conditions can be for instance at room temperature for 2 hours by using a buffer containing 25% formamide, 4x SSC, 50 mM NaH2PO4 / Na2HPO4 buffer; pH 7.0,5x Denhardt's, 1 mM EDTA,1 mg/ml DNA + 20 to 200 ng/ml probe to be tested (approx. 20 - 200 ng/ml)). This can be also predicted by standard calculation of hybridization using the number of complementary bases within the sequence and the content in G-C at room temperature as provided in the literature. Preferentially, the sequences are complementary to each other pursuant to the complementarity between two nucleic acid strands relying on Watson-Crick base pairing between the strands, i.e. the inherent base pairing between adenine and thymine (A-T) nucleotides and guanine and cytosine (G-C) nucleotides. Accurate base pairing equates with Watson-Crick base pairing includes base pairing between standard and modified nucleosides and base pairing between modified nucleosides, where the modified nucleosides are capable of substituting for the appropriate standard nucleosides according to the Watson-Crick pairing. The complementary sequence of the single-strand oligonucleotide can be any length that supports specific and stable hybridization between the two single-strand oligonucleotides under the reaction conditions. The complementary sequence generally authorizes a partial double stranded overlap between the two hybridized oligonucleotides over more than 3bp, preferably more than 5 bp, preferably more than to 10 bp. The complementary sequence is advantageously selected not to be homologous to any sequence in the genome to avoid off-target recombination or recombination not involving the whole donor matrix (i.e. only one oligonucleotide).

By "nucleic acid homologous sequence" it is meant a nucleic acid sequence with enough identity to another one to lead to homologous recombination between sequences, more particularly having at least 80% identity, preferably at least 90% identity and more preferably at least 95%, and even more preferably 98 % identity. "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting.

- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting.

The terms "vector" or "vectors" refer to a nucleic acid molecule capable of transporting another nucleic acid to which it has been linked. A "vector" in the present invention includes, but is not limited to, a viral vector, a plasmid, a RNA vector or a linear or circular DNA or RNA molecule which may consists of a chromosomal, non-chromosomal, semi-synthetic or synthetic nucleic acids. Preferred vectors are those capable of autonomous replication (episomal vector) and/or expression of nucleic acids to which they are linked (expression vectors). Large numbers of suitable vectors are known to those of skill in the art and commercially available. Viral vectors include retrovirus, adenovirus, parvovirus (e. g. adenoassociated viruses), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example. Examples of retroviruses include: avian leukosis-sarcoma, mammalian C-type, B-type viruses, D type viruses, HTLV-BLV group, lentivirus, spumavirus (Coffin, J. M., Retroviridae: The viruses and their replication, In Fundamental Virology, Third Edition, B. N. Fields, et al., Eds., Lippincott-Raven Publishers, Philadelphia, 1996).
Azencott, H. R., G. F. Peter, et al. (2007). "Influence of the cell wall on intracellular delivery to algal cells by electroporation and sonication." Ultrasound Med Biol 33(11): 1805-17.
Bernard, P., P. Gabant, et al. (1994). "Positive-selection vectors using the F plasmid ccdB killer gene." Gene 148(1): 71-4.
Bochner, B. R., H. C. Huang, et al. (1980). "Positive selection for loss of tetracycline resistance." J Bacteriol 143(2): 926-33.
Collier, D. N., C. Spence, et al. (2001). "Isolation and phenotypic characterization of Pseudomonas aeruginosa pseudorevertants containing suppressors of the catabolite repression control-defective crc-10 allele." FEMS Microbiol Lett 196(2): 87-92.
Cong, L., F. A. Ran, et al. (2013). "Multiplex genome engineering using CRISPR/Cas systems." Science 339(6121): 819-23.
Critchlow, S. E. and S. P. Jackson (1998). "DNA end-joining: from yeast to man." Trends Biochem Sci 23(10): 394-8.
Daboussi, M. J., A. Djeballi, et al. (1989). "Transformation of seven species of filamentous fungi using the nitrate reductase gene of Aspergillus nidulans." Curr Genet 15(6): 453-6.
Dalgaard, J. Z., A. J. Klar, et al. (1997). "Statistical modeling and analysis of the LAGLIDADG family of site-specific endonucleases and identification of an intein that encodes a site-specific endonuclease of the HNH family." Nucleic Acids Res 25(22): 4626-38.
De Riso, V., R. Raniello, et al. (2009). "Gene silencing in the marine diatom Phaeodactylum tricornutum." Nucleic Acids Res 37(14): e96.
Dean, D. (1981). "A plasmid cloning vector for the direct selection of strains carrying recombinant plasmids." Gene 15(1): 99-102.
Deltcheva, E., K. Chylinski, et al. (2011). "CRISPR RNA maturation by trans-encoded small RNA and host factor RNase III." Nature 471(7340): 602-7.
Deveau, H., R. Barrangou, et al. (2008). "Phage response to CRISPR-encoded resistance in Streptococcus thermophilus." J Bacteriol 190(4): 1390-400.
Falciatore, A., L. Merendino, et al. (2005). "The FLP proteins act as regulators of chlorophyll synthesis in response to light and plastid signals in Chlamydomonas." Genes Dev 19(1): 176-87.
Gasiunas, G., R. Barrangou, et al. (2012). "Cas9-crRNA ribonucleoprotein complex mediates specific DNA cleavage for adaptive immunity in bacteria." Proc Natl Acad Sci U S A 109(39): E2579-86.
Gay, P., D. Le Coq, et al. (1985). "Positive selection procedure for entrapment of insertion sequence elements in gram-negative bacteria." J Bacteriol 164(2): 918-21.
Gorbalenya, A. E. (1994). "Self-splicing group I and group II introns encode homologous (putative) DNA endonucleases of a new family." Protein Sci 3(7): 1117-20.
Haft, D. H., J. Selengut, et al. (2005). "A guild of 45 CRISPR-associated (Cas) protein families and multiple CRISPR/Cas subtypes exist in prokaryotic genomes." PLoS Comput Biol 1(6): e60.
Horvath, P. and R. Barrangou (2010). "CRISPR/Cas, the immune system of bacteria and archaea." Science 327(5962): 167-70.
Jinek, M., K. Chylinski, et al. (2012). "A programmable dual-RNA-guided DNA endonuclease in adaptive bacterial immunity." Science 337(6096): 816-21.
Kast, P. (1994). "pKSS--a second-generation general purpose cloning vector for efficient positive selection of recombinant clones." Gene 138(1-2): 109-14.
Kilian, O., C. S. Benemann, et al. (2011). "High-efficiency homologous recombination in the oil-producing alga Nannochloropsis sp." Proc Natl Acad Sci U S A 108(52): 21265-9.
Kleanthous, C., U. C. Kuhlmann, et al. (1999). "Structural and mechanistic basis of immunity toward endonuclease colicins." Nat Struct Biol 6(3): 243-52.
Ma, J. L., E. M. Kim, et al. (2003). "Yeast Mre11 and Rad1 proteins define a Ku-independent mechanism to repair double-strand breaks lacking overlapping end sequences." Mol Cell Biol 23(23): 8820-8.
Makarova, K. S., N. V. Grishin, et al. (2006). "A putative RNA-interference-based immune system in prokaryotes: computational analysis of the predicted enzymatic machinery, functional analogies with eukaryotic RNAi, and hypothetical mechanisms of action." Biol Direct 1: 7.
Mali, P., L. Yang, et al. (2013). "RNA-guided human genome engineering via Cas9." Science 339(6121): 823-6.
Maliga, P. (2004). "Plastid transformation in higher plants." Annu Rev Plant Biol 55: 289-313.
Maloy, S. R. and W. D. Nunn (1981). "Selection for loss of tetracycline resistance by Escherichia coli." J Bacteriol 145(2): 1110-1.
Mojica, F. J., C. Diez-Villasenor, et al. (2009). "Short motif sequences determine the targets of the prokaryotic CRISPR defence system." Microbiology 155(Pt 3): 733-40.
Murphy, C. K., E. J. Stewart, et al. (1995). "A double counter-selection system for the study of null alleles of essential genes in Escherichia coli." Gene 155(1): 1-7.
Pooga, M. and U. Langel (2005). "Synthesis of cell-penetrating peptides for cargo delivery." Methods Mol Biol 298: 77-89.
Qi, L. S., M. H. Larson, et al. (2013). "Repurposing CRISPR as an RNA-guided platform for sequence-specific control of gene expression." Cell 152(5): 1173-83.
Rohr, J., N. Sarkar, et al. (2004). "Tandem inverted repeat system for selection of effective transgenic RNAi strains in Chlamydomonas." Plant J 40(4): 611-21.
Sakaguchi, T., K. Nakajima, et al. (2011). "Identification of the UMP synthase gene by establishment of uracil auxotrophic mutants and the phenotypic complementation system in the marine diatom Phaeodactylum tricornutum." Plant Physiol 156(1): 78-89.
Sapranauskas, R., G. Gasiunas, et al. (2011). "The Streptococcus thermophilus CRISPR/Cas system provides immunity in Escherichia coli." Nucleic Acids Res 39(21): 9275-82.
Schutz, K., J. R. Hesselberth, et al. (2010). "Capture and sequence analysis of RNAs with terminal 2',3'-cyclic phosphates." Rna 16(3): 621-31.
Shub, D. A., H. Goodrich-Blair, et al. (1994). "Amino acid sequence motif of group I intron endonucleases is conserved in open reading frames of group II introns." Trends Biochem Sci 19(10): 402-4.
Siaut, M., M. Heijde, et al. (2007). "Molecular toolbox for studying diatom biology in Phaeodactylum tricornutum." Gene 406(1-2): 23-35.
Stacey, K. A. and E. Simson (1965). "Improved Method for the Isolation of Thymine-Requiring Mutants of Escherichia Coli." J Bacteriol 90: 554-5.
Steinmetz, M., D. Le Coq, et al. (1983). "[Genetic analysis of sacB, the structural gene of a secreted enzyme, levansucrase of Bacillus subtilis Marburg]." Mol Gen Genet 191(1): 138-44.
van der Ploeg, J. R. (2009). "Analysis of CRISPR in Streptococcus mutans suggests frequent occurrence of acquired immunity against infection by M102-like bacteriophages." Microbiology 155(Pt 6): 1966-76.

## Claims

1. A method of genome engineering a diatom comprising:
(a) Selecting a target nucleic acid sequence, optionally comprising a PAM motif;
(b) Providing a diatom, wherein a transgene encoding Cas9 or at least one split Cas9 is stably integrated within the genome of said diatom;
(c) Providing at least one guide RNA comprising a complementary sequence to the target nucleic acid;
(d) Introducing into said diatom, at least one guide RNA such that said Cas9 or split Cas9 processes said target nucleic acid sequence;
wherein said guide RNA is fused to a cell-penetrating peptide, and said guide RNA is introduced into said diatom by contacting said diatom with the fusion guide RNA: cell-penetrating peptide.

2. The method of claim 1 wherein said Cas9 or split Cas9 is capable of cleaving said target nucleic acid sequence.

3. The method of claim 1 or 2 further comprising introducing into said diatom an exogenous nucleic acid comprising at least one a sequence homologous to a region of the target nucleic acid sequence such that homologous recombination occurs between the target nucleic acid sequence and the exogenous nucleic acid.

4. The method according to any one of claims 1 to 3 comprising the step of stably integrating said transgene encoding Cas9 or split Cas9 within the genome of the diatom.

5. The method of claim 1 further comprising selecting diatom comprising cell penetrating-peptide.

6. The method of claim 5 wherein said cell-penetrating peptide is fused to a reporter marker such as fluorescent protein or a tag marker.

7. The method according to claim 1, wherein said cell-penetrating peptide is fused to said guide RNA covalently.

8. The method of claim 7 wherein said cell-penetrating peptide is fused to said guide RNA by a disulfide bond.

9. The method according to claim 1, wherein said cell-penetrating peptide is fused to said guide RNA non-covalently.

10. The method according to any one of claims 1 to 9 wherein said cell-penetrating peptide is selected from the group consisting of: penetratin, TAT, polyarginine peptide, pVEC, MPG, Transportan, Guanidium rich molecular transporter.

11. The method according to any one of claims 1 to 10 wherein said Cell-penetrating peptide is fused to a cationic or liposomal polymer.

12. The method according to any one of claims 1 to 11 wherein said target nucleic acid sequence is a selectable marker gene.

13. The method according to any one of claims 1 to 12 wherein said diatoms are *Thalassiosira pseudonana* or *Phaedodactylum tricornutum.*

14. A diatom cell obtained by the method according to any one of claims 1 to 13.

15. A diatom cell comprising a Cas9 transgene stably integrated within the genome.

16. A diatom cell comprising a cell penetrating peptide fused to a guide RNA, which comprises a complementary sequence to a target nucleic acid such that, CAS9 or split CAS9 processes said target nucleic acid.

## Patentansprüche

1. Verfahren zur Genommanipulation einer Diatomee, umfassend:
(a) Auswählen einer Zielnukleinsäuresequenz, die gegebenenfalls ein PAM-Motiv umfasst;
(b) Bereitstellen einer Diatomee, wobei ein Transgen, das Cas9 oder mindestens ein Split-Cas9 kodiert, stabil innerhalb des Genoms der Diatomee integriert ist;
(c) Bereitstellen von mindestens einer Guide-RNA, die eine komplementäre Sequenz zur Zielnukleinsäure umfasst;
(d) Einführen in die Diatomee mindestens einer Guide-RNA so, dass das Cas9 oder Split-Cas9 die Zielnukleinsäuresequenz prozessiert;
wobei die Guide-RNA mit einem zellpenetrierenden Peptid fusioniert ist, und die Guide-RNA in die Diatomee durch Inkontaktbringen der Diatomee mit der Fusion Guide-RNA: zellpenetrierendes Peptid eingeführt wird.

2. Verfahren nach Anspruch 1, wobei das Cas9 oder Split-Cas9 zum Spalten der Zielnukleinsäuresequenz fähig ist.

3. Verfahren nach Anspruch 1 oder 2, das weiterhin Einführen in die Diatomee einer exogenen Nukleinsäure umfasst, die mindestens eine Sequenz umfasst, die homolog zu einer Region der Zielnukleinsäuresequenz ist, so dass homologe Rekombination zwischen der Zielnukleinsäuresequenz und der exogenen Nukleinsäure auftritt.

4. Verfahren nach einem beliebigen der Ansprüche 1 bis 3, das den Schritt des stabilen Integrierens des Transgens, das Cas9 oder Split-Cas9 kodiert, innerhalb des Genoms der Diatomee umfasst.

5. Verfahren nach Anspruch 1, das weiterhin Auswählen einer Diatomee umfasst, die ein zellpenetrierendes Peptid umfasst.

6. Verfahren nach Anspruch 5, wobei das zellpenetrierende Peptid mit einem Reportermarker wie einem fluoreszierenden Protein oder einem Tag-Marker fusioniert ist.

7. Verfahren nach Anspruch 1, wobei das zellpenetrierende Peptid kovalent mit der Guide-RNA fusioniert ist.

8. Verfahren nach Anspruch 7, wobei das zellpenetrierende Peptid mit der Guide-RNA durch eine Disulfidbindung fusioniert ist.

9. Verfahren nach Anspruch 1, wobei das zellpenetrierende Peptid nicht kovalent mit der Guide-RNA fusioniert ist.

10. Verfahren nach einem beliebigen der Ansprüche 1 bis 9, wobei das zellpenetrierende Peptid aus der Gruppe bestehend aus: Penetratin, TAT, Polyargininpeptid, pVEC, MPG, Transportan, guanidiniumreicher molekularer Transporter ausgewählt ist.

11. Verfahren nach einem beliebigen der Ansprüche 1 bis 10, wobei das zellpenetrierende Peptid mit einem kationischen oder liposomalen Polymer fusioniert ist.

12. Verfahren nach einem beliebigen der Ansprüche 1 bis 11, wobei die Zielnukleinsäuresequenz ein Selektionsmarkergen ist.

13. Verfahren nach einem beliebigen der Ansprüche 1 bis 12, wobei die Diatomeen *Thalassiosira pseudonana* oder *Phaedodactylum tricornutum* sind.

14. Ditomeenzelle, erhalten durch das Verfahren gemäß einem beliebigen der Ansprüche 1 bis 13.

15. Ditomeenzelle, die ein Cas9-Transgen stabil innerhalb des Genoms integriert umfasst.

16. Ditomeenzelle, die ein zellpenetrierendes Peptid mit einer Guide-RNA fusioniert umfasst, welche eine komplementäre Sequenz zu einer Zielnukleinsäure so umfasst, dass Cas9 oder Split-Cas9 die Zielnukleinsäure prozessiert.

## Revendications

1. Procédé de génie génomique d'une diatomée comprenant :
(a) la sélection d'une séquence d'acide nucléique cible, comprenant éventuellement un motif PAM ;
(b) la fourniture d'une diatomée, dans laquelle un transgène codant pour Cas9 ou au moins une Cas9 scindée est intégré de manière stable dans le génome de ladite diatomée ;
(c) la fourniture d'au moins un ARN guide comprenant une séquence complémentaire de l'acide nucléique cible ;
(d) l'introduction dans ladite diatomée, d'au moins un ARN guide de sorte que ladite Cas9 ou ladite Cas9 scindée traite ladite séquence d'acide nucléique cible ;
dans lequel ledit ARN guide est fusionné à un peptide de pénétration cellulaire, et ledit ARN guide est introduit dans ladite diatomée par mise en contact de ladite diatomée avec la fusion d'ARN guide:peptide de pénétration cellulaire.

2. Procédé selon la revendication 1, dans lequel ladite Cas9 ou Cas9 scindée est apte à cliver ladite séquence d'acide nucléique cible.

3. Procédé selon la revendication 1 ou 2, comprenant en outre l'introduction dans ladite diatomée d'un acide nucléique exogène comprenant au moins une séquence homologue à une région de la séquence d'acide nucléique cible de sorte qu'une recombinaison homologue se produit entre la séquence d'acide nucléique cible et l'acide nucléique exogène.

4. Procédé selon l'une quelconque des revendications 1 à 3 comprenant l'étape d'intégration de manière stable dudit transgène codant pour Cas9 ou une Cas9 scindée dans le génome de la diatomée.

5. Procédé selon la revendication 1, comprenant en outre la sélection d'une diatomée comprenant un peptide de pénétration cellulaire.

6. Procédé selon la revendication 5, dans lequel ledit peptide de pénétration cellulaire est fusionné à un marqueur rapporteur tel qu'une protéine fluorescente ou une étiquette marqueur.

7. Procédé selon la revendication 1, dans lequel ledit peptide de pénétration cellulaire est fusionné audit ARN guide de manière covalente.

8. Procédé selon la revendication 7, dans lequel ledit peptide de pénétration cellulaire est fusionné audit ARN guide par une liaison disulfure.

9. Procédé selon la revendication 1, dans lequel ledit peptide de pénétration cellulaire est fusionné audit ARN guide de manière non covalente.

10. Procédé selon l'une quelconque des revendications 1 à 9, dans lequel ledit peptide de pénétration cellulaire est choisi dans le groupe constitué par : la pénétratine, TAT, un peptide polyarginine, pVEC, MPG, le Transportan, un transporteur moléculaire riche en guanidium.

11. Procédé selon l'une quelconque des revendications 1 à 10, dans lequel ledit peptide de pénétration cellulaire est fusionné à un polymère cationique ou liposomique.

12. Procédé selon l'une quelconque des revendications 1 à 11, dans lequel ladite séquence d'acide nucléique cible est un gène marqueur de sélection.

13. Procédé selon l'une quelconque des revendications 1 à 12, dans lequel lesdites diatomées sont *Thalassiosira pseudonana* ou *Phaedodactylum tricornutum.*

14. Cellule de diatomée obtenue par le procédé selon l'une quelconque des revendications 1 à 13.

15. Cellule de diatomée comprenant un transgène de Cas9 intégré de manière stable dans le génome.

16. Cellule de diatomée comprenant un peptide de pénétration cellulaire fusionné à un ARN guide, qui comprend une séquence complémentaire d'un acide nucléique cible de sorte que, CAS9 ou une CAS9 scindée traite ledit acide nucléique cible.
